# FASCICULE DE BREVET EUROPEEN

(11) **EP 1 156 786 B1**
(45) Date de publication et mention de la délivrance du brevet: **26.03.2003**
(21) Numéro de dépôt: 00907743.9
(22) Date de dépôt: 29.02.2000
(51) Int. Cl.: A61K 9/20

(54) **COMPRIME ORODISPERSIBLE PRESENTANT UNE FAIBLE FRIABILITE ET SON PROCEDE DE PREPARATION**
IN DER MUNDHÖHLE DISPERGIERBARE TABLETTE MIT GERINGER BRÜCHIGKEIT SOWIE VERFAHREN ZU DEREN HERSTELLUNG
ORALLY DISPERSIBLE TABLET WITH LOW FRIABILITY AND METHOD FOR PREPARING SAME

(30) Priorité: 01.03.1999 FR 9902516
(43) Date de publication de la demande: 28.11.2001
(73) Titulaire: ETHYPHARM, 78550 Houdan (FR)
(72) Inventeur: DI COSTANZO, Laurent, F-94120 Fontenay-sous-Bois (FR); GENDROT, Edouard, André, F-28500 Garnay (FR); DI COSTANZO, Mathieu, Ernest, Jean-Baptiste, F-06650 Opio (FR); CHAUVEAU, Charles, André, F-06560 Valbonne (FR)
(74) Mandataire: Touati, Catherine
(86) Numéro de dépôt international: FR0000495
(87) Numéro de publication internationale: WO00051568

(56) Documents cités:
- EP-A- 0 676 280
- EP-A- 0 745 382
- DE-A- 4 318 577
- FR-A- 2 766 089

## Description

L'invention a pour objet un comprimé orodispersible, c'est à dire un comprimé à délitement rapide du genre de ceux qui sont destinés à se désagréger dans la bouche au contact de la salive préférentiellement en moins de 40 secondes et même en moins de 30 secondes. Elle vise également le procédé de préparation de ce comprimé.

Les comprimés à délitement rapide déjà connus, par exemple ceux décrits par la Société Demanderesse dans FR 97 09233, FR 98 14034 , FR 92 08642 et FR 91 09245 présentent souvent une friabilité élevée ce qui impose des précautions particulières lors des opérations de transfert et de conditionnement et limite le choix du type de conditionnement utilisé.

L'invention a pour but, surtout, de fournir des comprimés du genre en question présentant une palatabilité agréable et une friabilité (mesurée comme indiqué dans la pharmacopée française (Xème édition, "V.5.1-friabilité des comprimés", janvier 1993), c'est-à-dire une dureté et une résistance à l'abrasion, permettant leur conditionnement et leur transport par des moyens classiques ainsi qu'une utilisation facile pour le patient.

La Société Demanderesse a trouvé que de façon surprenante et inattendue, il était possible de faire comporter l'ensemble de ces propriétés, dont certaines peuvent paraître incompatibles avec d'autres, à un comprimé à délitement rapide du genre de ceux qui sont destinés à se désagréger dans la bouche en moins de 30 secondes au contact de la salive en formant une suspension aisée à avaler, lesdits comprimés étant à base d'une matière active sous forme de microcristaux ou microgranules enrobés et d'un mélange d'excipients comprenant au moins un agent de désagrégation, un agent soluble et un agent lubrifiant, dès lors qu'avant compression au moins la majeure partie de l'agent lubrifiant n'est plus incorporée dans le mélange d'excipients mais placée au contact de la surface extérieure de la masse constitutive du futur comprimé.

Un tel comprimé peut être conditionné au moyen de procédés standard, c'est-à-dire en utilisant des dispositifs industriels classiques. Le comprimé est suffisamment dur pour permettre une extraction aisée hors du blister dans lequel il est conditionné, cette extraction étant opérée par déchirure, perforation ou rupture de l'opercule de celui-ci sous la poussée du comprimé avec un risque substantiellement réduit de cassure.

Le comprimé conforme à l'invention est donc caractérisé par le fait que l'agent lubrifiant qui entre dans sa constitution et qui est sous forme pulvérulente se trouve réparti pour au moins sa majeure partie à la surface du comprimé, et par le fait que sa friabilité, mesurée comme indiqué dans la pharmacopée française (Xème édition, "V.5.1-friabilité des comprimés", janvier 1993), est inférieure à 1%, de préférence à 0,5%.

L'agent lubrifiant est choisi parmi les agents lubrifiants pharmaceutiquement acceptables qui ont un point de fusion au moins égal à 35°C, de préférence supérieur à 50°C.

De préférence, l'agent lubrifiant est choisi dans le groupe comprenant notamment le stéarate de magnésium, le stéarylfumarate de sodium, l'acide stéarique, le polyoxyéthylèneglycol micronisé (macrogol 6000 micronisé), la leucine, le benzoate de sodium et leurs mélanges.

La quantité d'agent lubrifiant, mise en oeuvre dans le comprimé conforme à l'invention, est de 0,2 à 10 pour mille (poids de l'agent lubrifiant/poids total du comprimé), de préférence de l'ordre de 3 à 6 pour mille.

Selon un mode de réalisation avantageux du comprimé selon l'invention, la totalité de l'agent lubrifiant est répartie à la surface extérieure du comprimé.

Il est souligné que cette quantité est jusqu'à dix fois inférieure à celle qui était nécessaire dans les comprimés à délitement rapide du genre en question connus dans lesquels l'agent lubrifiant est réparti au sein de l'excipient.

La granulométrie de l'agent lubrifiant pulvérulent est telle que lorsqu'il est projeté contre une surface, ses particules constitutives y adhèrent.

Selon un mode de réalisation avantageux, cette granulométrie est inférieure à 30 microns et de préférence inférieure à 10 microns.

L'agent de désagrégation est choisi dans le groupe comprenant notamment la carboxyméthylcellulose sodique réticulée désignée dans le métier par le terme croscarmellose, la crospovidone et leurs mélanges.

L'agent soluble est de préférence un agent soluble diluant à propriétés liantes, tel que notamment un polyol. Cet agent soluble peut de façon avantageuse être choisi conformément à ce qui est décrit dans les demandes de brevet au nom de la Société Demanderesse FR9709233 ou FR9814034.

Selon un mode de réalisation avantageux du comprimé conforme à l'invention, le mélange d'excipients comporte un agent perméabilisant, un agent solubilisant, des édulcorants, des arômes et des colorants.

Comme agent perméabilisant, on peut utiliser un composé choisi dans le groupe comprenant notamment les silices ayant une grande affinité pour les solvants aqueux, tels que la silice précipitée, plus connue sous le nom de marque SYLOID®, la silice colloïdale plus connue sous le nom d'AEROSIL® 200, les maltodextrines, les bétacyclodextrines et leurs mélanges.

L'édulcorant peut être choisi dans le groupe comprenant notamment l'aspartame, l'acésulfame de potassium, le saccharinate de sodium, la néohespéridine didrochalcone et leurs mélanges.

Les arômes et colorants sont ceux utilisés habituellement en pharmacie pour la préparation des comprimés.

Toute substance active pouvant être mis en oeuvre dans des comprimés à délitement rapide du genre en question peut avantageusement être utilisée dans les comprimés conformes à l'invention.

A titre de substance active, on peut utiliser au moins l'une de celles du groupe comprenant les sédatifs gastro-intestinaux, les antiacides, les antalgiques, les anti- inflammatoires, les vasodilatateurs coronariens, les vasodilatateurs périphériques et cérébraux, les antiinfectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

Pour des comprimés de 17mm de diamètre conformes à l'invention, la dureté est de façon avantageuse supérieure à 20 N, de préférence supérieure à 40 N et plus préférentiellement encore supérieure à 80 N. Cette dureté est dans tous les cas au moins égale à la force nécessaire pour rompre l'opercule fermant le blister dans lequel le comprimé est conditionné.

La friabilité des comprimés conformes à l'invention mesurée selon le procédé décrit dans la pharmacopée française est inférieure à 1%, de préférence à 0,5%.

La dimension la plus grande du comprimé conforme à l'invention peut être supérieure à 5 mm, voire à 17 mm et peut atteindre 25 mm.

Les comprimés classiques de cette taille ont tendance à se rompre lorsque, conditionnés dans des blisters, on les en retire en vue de leur administration, notamment lorsque le blister est en totalité constitué d'une matière métallique telle que l'aluminium.

Grâce à leur faible friabilité, de telles ruptures ne se produisent pas dans le cas des comprimés conformes à l'invention, qui sont donc tout particulièrement adaptés à être conditionnés dans des blisters constitués en totalité d'aluminium.

En effet, la bonne résistance à la rupture des comprimés selon l'invention permet de réduire substantiellement les risques de cassure du comprimé et permet une extraction aisée du comprimé hors du blister par déchirure, perforation ou rupture de l'opercule de celui-ci sous la poussée du comprimé conforme à l'invention.

Par ailleurs, le comprimé conforme à l'invention permet de satisfaire les normes de sécurité vis-à-vis des enfants, étant donné qu'il peut être conservé dans des blisters à double protection, c'est-à-dire déchirables et/ou pelables, le risque de cassure lors de l'extraction hors d'un conditionnement constitué par blister non pelable est substantiellement réduit.

Il est donc possible de conditionner les comprimés conformes à l'invention dans des blisters tout en aluminium d'une épaisseur importante permettant une totale étanchéité à l'humidité et donc d'obtenir un produit commercial présentant d'excellentes propriétés de conservation.

S'agissant de préparer le comprimé conforme à l'invention, conformément à l'invention on procède comme indiqué ci-après.

On connaît déjà des procédés de préparation de comprimés de composition classique comportant nécessairement en mélange avec leurs autres constituants, les quantités habituelles et importantes d'agent lubrifiant, représentant généralement de 0,5 à 2 % du poids du comprimé et permettant non seulement de favoriser la compression mais également de faciliter l'écoulement du mélange pulvérulent ; ces procédés mettent en oeuvre des dispositifs, tels que celui décrit dans le brevet EP 673 280, propres à pulvériser du lubrifiant sur les matrices de machines à comprimer pour limiter ou empêcher le grippage des machines à comprimer.

Les comprimés obtenus par ces procédés ne présentent pas les propriétés avantageuses dont il a été question plus haut des comprimés conformes à l'invention.

Ces derniers peuvent être obtenus par mise en oeuvre du procédé conforme à l'invention qui comprend successivement:
- la sélection, d'une part, d'une substance active sous forme de microcristaux ou microgranules enrobés, et, d'autre part, d'un ensemble d'excipients comprenant un agent de désagrégation, un agent soluble, ainsi qu'un agent lubrifiant ;
- le mélange de la substance active et des excipients sauf au moins la plus grande partie de l'agent lubrifiant ;
- l'introduction d'une quantité de ce mélange nécessaire à la constitution d'un comprimé dans la cavité d'un dispositif de compression à l'intérieur de laquelle le mélange doit être comprimé et sur les parois de laquelle a été appliquée préalablement la quantité nécessaire d'agent lubrifiant ;
- la compression du mélange et l'éjection du comprimé formé.

Le procédé conforme à l'invention présente l'avantage résidant dans le fait que les forces de compression devant être appliquées pour l'obtention du comprimé sont sensiblement plus faibles que celles utilisées dans les procédés connus, tout en conduisant à une dureté égale, voire supérieure à celle des comprimés classiques.

Selon un mode de réalisation avantageux du procédé conforme à l'invention, les forces de compression sont de 3 à 50kN, de préférence de 4 à 40kN et plus préférentiellement encore de 5 à 25kN.

Même avec de telles forces de compression, il est possible d'obtenir des comprimés de grande dimension présentant une dureté supérieure à 20 N, de préférence supérieure à 40 N, et plus préférentiellement encore supérieure à 80 N.

On rappelle par ailleurs, qu'avec les comprimés de l'art antérieur, il était nécessaire de modifier la quantité de lubrifiant incorporé dans le mélange d'excipients en fonction du principe actif mis en oeuvre dans le comprimé. Au contraire et de façon tout à fait avantageuse, le procédé conforme à l'invention ne requiert pas une telle modification de la formulation du mélange d'excipients en fonction du principe actif mis en oeuvre.

L'invention pourra être mieux comprise à l'aide des exemples non limitatifs qui suivent et qui sont relatifs à des modes de réalisation avantageux.

### EXEMPLE 1 :

### Comprimé de paracétamol dosé à 500 mg.

Le Tableau 1 donne la formule unitaire et la formule centésimale de ce comprimé.

**TABLEAU 1**

| **CONSTITUANTS** | **FORMULE UNITAIRE** | **FORMULE CENTESIMALE** |
|---|---|---|
| Paracétamol enrobé | 548,70 | 39,17 |
| Mannitol pour compression directe | 514,80 | 36,74 |
| Mannitol poudre cristalline | 171,50 | 12,24 |
| Crospovidone | 120,00 | 8,57 |
| Aspartam | 40,00 | 2,86 |
| Arôme cassis | 5,00 | 0,36 |
| Stéarate de Magnésium | 0,90 | 0,06 |
| **TOTAL** | **1400,70mg** | **100,0 %** |

Ce comprimé est préparé comme indiqué ci-après.

On introduit les microcristaux de paracétamol dans une installation à lit fluidisé et on pulvérise sur les microcristaux une dispersion dans l'éthanol d'EUDRAGIT E 100, d'EUDRAGIT NE 30 D et de silice colloïdale de façon à obtenir des microcristaux enrobés avec 10% de polymère ayant la formule donnée dans le tableau 2 ci-dessous.

On tamise tous les excipients sauf le stéarate de magnésium et on homogénéise le mélange comprenant le paracétamol enrobé et les excipients dans un mélangeur à sec.

On procède à la compression avec une comprimeuse équipée de matrices et de poinçons de diamètre 17 mm ; on pulvérise préalablement sur les parois des matrices et sur les poinçons du stéarate de magnésium en tant que lubrifiant (la quantité excédentaire non adhérente de stéarate de magnésium étant aspirée avant la compression).

La force de compression est de l'ordre de 16kN à 25kN ce qui permet d'obtenir des comprimés d'une dureté de 80 Newtons.

Le temps de désagrégation dans la bouche des comprimés ainsi obtenus est inférieur à 30 secondes.

Ce temps correspond à la durée qui sépare, d'une part, le moment de la mise en place du comprimé dans la bouche au contact de la salive et, d'autre part, le moment de la déglutition de la suspension résultant de la désagrégation du comprimé au contact de la salive.

La friabilité mesurée selon le procédé décrit dans la pharmacopée française (Xème édition, "V.5.1- friabilité des comprimés", Janvier 1993) à l'aide d'un appareil à ailettes est inférieure à 1%.

La quantité de stéarate de magnésium réparti à la surface d'un comprimé est de 0,9mg soit 0.64 pour mille.

**Tableau 2**

| **CONSTITUANTS** | **FORMULE UNITAIRE** | **FORMULE CENTESIMALE** |
|---|---|---|
| Paracétamol | 500,00 | 91,12 |
| Eudragit NE 30 D en sec | 12,10 | 2,21 |
| Eudragit E 100 | 24,30 | 4,43 |
| Silice colloïdale | 12,30 | 2,24 |
| **TOTAL** | **548,70 mg** | **100,0 %** |

### EXEMPLE 2 :

### Comprimé d'ibuprofène dosé à 200 mg.

Le tableau 3 donne la formule unitaire de ce comprimé.

**TABLEAU 3**

| **CONSTITUANTS** | **FORMULE UNITAIRE** | **FORMULE CENTESIMALE** |
|---|---|---|
| Granulé enrobé d'ibuprofène | 256,20 | 36,60 |
| Mannitol granulé | 192,80 | 27,54 |
| Mannitol poudre | 193,40 | 27,63 |
| Croscarmellose | 21,00 | 3,00 |
| Silice-précipitée | 7,00 | 1,00 |
| Aspartam | 25,00 | 3,57 |
| Arôme citron | 4,00 | 0,57 |
| Stéarate de magnésium | 0,60 | 0,09 |
| **TOTAL** | **700,00 mg** | **100,00** |

On calibre les excipients identifiés dans le tableau 2 sur une grille de 1000 microns d'ouverture de mailles.

On pèse les différents constituants dans des récipients séparés de contenance adaptée.

On introduit dans un mélangeur par retournement des particules enrobées d'ibuprofène (dont la formule est donnée dans le tableau 3 ci-après), le mannitol granulé, le mannitol pulvérulent, la croscarmellose, l'aspartam, la silice précipitée et l'arôme.

On prépare un mélange homogène.

On pulvérise sur les parois des matrices et sur les poinçons d'une machine rotative du stéarate de magnésium (on aspire la quantité excédentaire de stéarate de magnésium).

On comprime le mélange obtenu introduit dans les matrices de la machine rotative entre les poinçons recouverts de stéarate de magnésium avec une force de compression de l'ordre de 7 kN, afin d'obtenir des comprimés ayant les caractéristiques suivantes :
- masse moyenne comprise entre 665 mg et 735 mg ;
- résistance à la rupture comprise entre 20 et 50N;
- friabilité inférieure à 1% ;
- temps de désagrégation moyen en bouche inférieur à 30 secondes.

Ce temps de désagrégation correspond à la durée qui sépare, d'une part, le moment de la mise en place du comprimé dans la bouche au contact de la salive et, d'autre part, le moment de déglutition de la suspension résultant de la désagrégation du comprimé au contact de la salive.

La quantité de stéarate de magnésium sur le comprimé final est de 0,6mg soit 0,8 pour mille.

**TABLEAU 4**

| **Formule du granulé enrobé d'ibuprofène** | | |
|---|---|---|
| **CONSTITUANTS** | **FORMULE UNITAIRE** | **FORMULE CENTESIMALE** |
| Ibuprofène | 200,00 | 78,06 |
| Ethylcellulose | 35,00 | 13,66 |
| Silice précipitée | 14,20 | 5,55 |
| HPMC* | 7,00 | 2,73 |
| **TOTAL** | **256,20 mg** | **100,00 %** |

| | | |
|---|---|---|
| *HPMC : hydroxypropylméthylcellulose | | |

### EXEMPLE 3 :

### Comprimé d'aspirine dosé à 500 mg

Le tableau 5 donne la formule unitaire de ce comprimé.

**TABLEAU 5**

| **CONSTITUANTS** | **FORMULE UNITAIRE** | **FORMULE CENTESIMALE** |
|---|---|---|
| Granulé enrobé d'aspirine | 564,00 | 40,29 |
| Mannitol granulé, | 336,00 | 24,00 |
| Mannitol pulvérulent | 336,00 | 24,00 |
| Crospovidone | 120,00 | 8,57 |
| Silice précipitée | 14,00 | 1,00 |
| Aspartam | 14,40 | 1,03 |
| Acésulfame de potassium | 9,60 | 0,89 |
| Arôme citron | 5,00 | 0,36 |
| Stéarylfumarate de sodium | 0,90 | 0,06 |
| | **1400,00 mg** | **100,00 %** |

Les comprimés sont préparés de la même façon que dans l'exemple 1, à l'aide de granulés enrobés ayant la formule donnée dans le tableau 6 ci-après et par compression sur une comprimeuse dont les parois des matrices et les poinçons ont préalablement été recouverts par pulvérisation de stéarylfumarate de sodium.

**TABLEAU 6**

| **Formule du granulé enrobé d'aspirine** | | |
|---|---|---|
| **CONSTITUANTS** | **FORMULE UNITAIRE** | **FORMULE CENTESIMALE** |
| Aspirine | 500,00 | 88,85 |
| Ethylcellulose | 50,00 | 8,87 |
| HPMC* | 10,00 | 1,77 |
| Silice colloïdale | 4,00 | 0,71 |
| | **564,00 mg** | **100,00 %** |

| | | |
|---|---|---|
| *HPMC : hydroxypropylméthylcellulose | | |

Les comprimés ainsi obtenus présentent les caractéristiques suivantes :
- quantité de stéarylfumarate de sodium: 0,9mg soit 0,64 pour mille
- Résistance à la rupture : 30 à 60 N
- Friabilité : inférieure à 1%
- Temps de désagrégation inférieur à 30s.

## Revendications

1. Comprimé à délitement rapide du genre de ceux qui sont destinés à se désagréger dans la bouche au contact de la salive en moins de 30 secondes et en formant une suspension aisée à avaler et qui est à base d'une matière active sous forme de microcristaux ou microgranules enrobés et d'un mélange d'excipients comprenant au moins un agent de désagrégation, un agent soluble et un agent lubrifiant, **caractérisé par le fait que** l'agent lubrifiant est sous forme pulvérulente et se trouve réparti pour au moins sa majeure partie à la surface du comprimé et que sa friabilité, mesurée comme indiqué dans la pharmacopée française (Xème édition, "V.5.1-friabilité des comprimés", janvier 1993), est inférieure à 1%, de préférence inférieure à 0,5%, ce grâce à quoi ledit comprimé peut être conditionné au moyen de procédés standard et présente une dureté nécessaire et suffisante pour permettre son extraction aisée hors du blister dans lequel il est conditionné par perforation de l'opercule de celui-ci sous la poussée du comprimé avec un risque substantiellement réduit de cassure lors de l'extraction.

2. Comprimé selon la revendication 1, **caractérisé par le fait que** sa plus grande dimension est supérieure à 5 mm, de préférence supérieure à 17 mm et peut atteindre 25 mm.

3. Comprimé selon la revendication 1 ou la revendication 2, **caractérisé par le fait que** l'agent lubrifiant est choisi parmi les agents lubrifiants pharmaceutiquement acceptables qui ont un point de fusion au moins égal à 35°C, de préférence supérieur à 50°C.

4. Comprimé selon l'une quelconque des revendications 1 à 3, **caractérisé par le fait que** le lubrifiant est choisi dans le groupe comprenant le stéarate de magnésium, le stéarylfumarate de sodium, l'acide stéarique et le polyoxyéthylène glycol micronisé.

5. Comprimé selon l'une quelconque des revendications 1 à 4, **caractérisé par le fait que** le lubrifiant est le stéarate de magnésium.

6. Comprimé selon l'une quelconque des revendications 1 à 5, **caractérisé par le fait que** la quantité d'agent lubrifiant est de l'ordre de 0,2 à 10 pour mille (poids de l'agent lubrifiant/poids total du comprimé), de préférence de l'ordre de 3 à 6 pour mille (poids de l'agent lubrifiant/poids total du comprimé).

7. Comprimé selon l'une quelconque des revendications 1 à 6, **caractérisé par le fait que** l'agent lubrifiant a une granulométrie telle que lorsqu'il est projeté contre une surface, ses particules constitutives y adhèrent, de préférence inférieure à 30 microns et plus préférentiellement encore inférieure à 10 microns.

8. Comprimé selon l'une quelconque des revendications 1 à 7, **caractérisé par le fait que** l'agent de désagrégation est choisi dans le groupe comprenant notamment la carboxyméthylcellulose sodique réticulée désignée dans le métier par le terme croscarmellose, la crospovidone et leurs mélanges.

9. Comprimé selon l'une quelconque des revendications 1 à 8, **caractérisé par le fait que** le mélange d'excipients peut comporter un agent perméabilisant, un agent solubilisant, des édulcorants, des arômes et des colorants.

10. Comprimé selon l'une quelconque des revendications 1 à 9, **caractérisé par le fait qu'**il est destiné à être conditionné dans des blisters totalement en aluminium, pouvant en outre comporter une pellicule de matière plastique à déchirer avant ouverture.

11. Procédé de préparation du comprimé selon l'une des revendications 1 à 10, **caractérisé par le fait qu'**il comprend successivement:
- la sélection, d'une part, d'une substance active sous forme de microcristaux ou microgranules enrobés, et, d'autre part, d'un ensemble d'excipients comprenant un agent de désagrégation, un agent soluble ainsi qu'un agent lubrifiant ;
- le mélange de la substance active et des excipients sauf au moins la plus grande partie de l'agent lubrifiant ;
- l'introduction d'une quantité de ce mélange nécessaire à la constitution d'un comprimé dans la cavité d'un dispositif de compression à l'intérieur de laquelle le mélange doit être comprimé et sur les parois de laquelle a été appliquée préalablement la quantité nécessaire d'agent lubrifiant ;
- la compression du mélange et l'éjection du comprimé formé.

12. Procédé selon la revendication 11, **caractérisé par le fait que** les forces de compression sont de 3 à 50kN, de préférence de 4 à 40kN et plus préférentiellement encore de 5 à 25kN.

## Claims

1. A rapidly disintegrating tablet similar to those designed to disintegrate in the mouth on contact with saliva in less than 30 seconds, forming an easy-to-swallow suspension, and based on an active substance in the form of coated microcrystals or microgranules, and a mixture of excipients including at least a disintegrating agent, a soluble agent and a lubricating agent, **characterized in that** the lubricating agent is in powder form and is distributed at least for the greater part on the tablet surface and that its friability, measured as specified in the French Pharmacopoeia (10th Edition, V.5.1 - Friability of Tablets, January 1993), is less than 1 %, and preferably less than 0.5 %, whereby said tablet can be packaged by standard processes and has the required and adequate hardness to enable it to be removed with ease from the blister pack in which it is packed, by perforating the seal thereof by pushing the tablet, with a substantially reduced risk of the tablet breaking during removal.

2. Tablet in accordance with Claim 1, **characterized in that** its largest dimension is greater than 5 mm, is preferably greater than 17 mm and may be as much as 25 mm.

3. Tablet in accordance with Claim 1 or Claim 2, **characterized in that** the lubricating agent is selected from among the pharmaceutically acceptable lubricating agents which have a melting point of at least 35°C, and preferably higher than 50°C.

4. Tablet in accordance with one of Claims 1 to 3, **characterized in that** the lubricating agent is selected from the group including magnesium stearate, sodium stearyl fumarate, stearic acid and micronized polyoxyethylene glycol.

5. Tablet in accordance with one of Claims 1 to 4, **characterized in that** the lubricating agent is magnesium stearate.

6. Tablet in accordance with one of Claims 1 to 5, **characterized in that** the quantity of lubricating agent is in the range 0.2 to 10 parts per 1000 (weight of lubricating agent / total weight of tablet), and is preferably in the range 3 to 6 parts per 1000 (weight of lubricating agent / total weight of tablet).

7. Tablet in accordance with one of Claims 1 to 6, **characterized in that** the lubricating agent has a particle size distribution such that its constituent particles adhere when it is sprayed against a surface, preferably less than 30 microns and more preferably still, less than 10 microns.

8. Tablet in accordance with one of Claims 1 to 7, **characterized in that** the disintegrating agent is selected from the group including in particular cross-linked sodium carboxymethylcellulose, known in the industry as croscarmellose, crospovidone and their mixtures.

9. Tablet in accordance with one of Claims 1 to 8, **characterized in that** the mixture of excipients may include a permeabilising agent, a solubilising agent, sweeteners, flavors and colorings.

10. Tablet in accordance with one of Claims 1 to 9, **characterized in that** it is designed to be packaged in blisters composed entirely of aluminum, which may in addition include a cover of a plastic material which is to be torn off before opening.

11. Process for the production of a tablet in accordance with one of Claims 1 to 10, **characterized in that** the process involves the following sequence of steps:
- choosing, firstly, an active substance in the form of coated microcrystals or microgranules, and secondly, a set of excipients including a disintegrating agent, a soluble agent, and also a lubricating agent;
- mixing the active substance and the excipients with the exception of at least the greater part of the lubricating agent;
- feeding a quantity of this mixture necessary to form a tablet into the cavity of a compression device within which the mixture is to be compressed and onto the walls of which the necessary quantity of lubricating agent has been applied in advance;
- compressing the mixture and ejecting the tablet formed.

12. Process in accordance with Claim 11, **characterized in that** the compression forces are in the range 3 kN to 50 kN, preferably in the range 4 kN to 40 kN, or more preferably still, in the range 5 kN to 25 kN.

## Patentansprüche

1. Tablette mit schnellem Zerfall von der Art, die dazu vorgesehen ist, sich im Mund in Kontakt mit dem Speichel in weniger als 30 Sekunden aufzulösen und eine Suspension zu bilden, die leicht hinuntergeschluckt werden kann, und die auf der Basis von einem Wirkstoff in Form von umhüllten Mikrokristallen oder Mikrokörnchen und einer Mischung von Füllstoffen besteht, die mindestens ein Zerfallsmittel, ein Lösliches Mittel und ein Gleitmittel umfaßt, **dadurch gekennzeichnet, daß** das Gleitmittel in Pulverform vorliegt und sich verteilt auf mindestens dem überwiegenden Teil der Oberfläche der Tablette befindet, und daß ihre Brüchigkeit, gemessen wie in dem französischen Arzneibuch (Xème édition, "V.5.1-friabilité des comprimés", janvier 1993) angegeben, unter 1 %, vorzugsweise unter 0,5 % liegt, aufgrund dessen die genannte Tablette mit Hilfe von Standardverfahren verpackt werden kann und Härte aufweist die erforderlich und ausreichend ist, um ihre leichte Entnahme aus dem Blister, in den sie verpackt wurde, durch Perforation seines Deckels unter dem Schub der Tablette zu ermöglichen, und zwar mit einem wesentlich verringerten Risiko des Zerbrechens bei der Entnahme.

2. Tablette nach Anspruch 1, **dadurch gekennzeichnet, daß** ihre größte Abmessung über 5 mm, vorzugsweise über 17 mm liegt und 25 mm erreichen kann.

3. Tablette nach Anspruch 1 oder Anspruch 2, **dadurch gekennzeichnet, daß** das Gleitmittel unter den pharmazeutisch akzeptablen Gleitmitteln ausgewählt wird, die einen Schmelzpunkt von mindestens gleich 35 °C, vorzugsweise von über 50°C besitzen.

4. Tablette nach irgendeinem der Ansprüche 1 bis 3, **dadurch gekennzeichnet, daß** das Gleitmittel aus der Gruppe gewählt wird, die Magnesiumstearat, Natriumstearylfumarat, Stearinsäure und mikronisiertes Polyoxyethylenglycol umfaßt.

5. Tablette nach irgendeinem der Ansprüche 1 bis 4, **dadurch gekennzeichnet, daß** das Gleitmittel Magnesiumstearat ist.

6. Tablette nach irgendeinem der Ansprüche 1 bis 5, **dadurch gekennzeichnet, daß** die Menge an Gleitmittel in der Größenordnung von 0,2 bis 10‰ (Gewicht des Gleitmittels/Gesamtgewicht der Tablette), vorzugsweise in der Größenordnung von 3 bis 6‰, (Gewicht des Gleitmittels/Gesamtgewicht der Tablette) liegt.

7. Tablette nach irgendeinem der Ansprüche 1 bis 6, **dadurch gekennzeichnet, daß** das Gleitmittel eine derartige Granulometrie besitzt, daß in dem Fall, wo es gegen eine Oberfläche geschleudert wird, seine Bestandteile daran haften bleiben, vorzugsweise unter 30 µm und noch bevorzugter von unter 10 µm.

8. Tablette nach irgendeinem der Ansprüche 1 bis 7, **dadurch gekennzeichnet, daß** das Zerfallsmittel aus der Gruppe gewählt wird, die insbesondere vernetzte Natriumcarboxymethylcellulose, im Fachjargon mit dem Ausdruck Croscarmellose bezeichnet, Crospovidon und deren Mischungen umfaßt.

9. Tablette nach irgendeinem der Ansprüche 1 bis 8, **dadurch gekennzeichnet, daß** die Mischung der Füllstoffe ein Permeabilisierungsmittel, einen Löslichkeitsvermittler, Süßstoffe, Aromen und Farbstoffe umfassen kann.

10. Tablette nach irgendeinem der Anssprüche 1 bis 9, **dadurch gekennzeichnet, daß** sie dazu vorgesehen ist, in vollständig aus Aluminium bestehenden Blistern konditioniert (verpackt) zu werden, die außerdem einen vor dem Öffnen aufzureißenden Film aus Kunststoff umfassen.

11. Verfahren zur Herstellung der Tablette nach einem der Ansprüche 1 bis 10, **dadurch gekennzeichnet, daß** es nacheinander umfaßt:
- die Selektion einerseits eines Wirkstoffes in Form von umhüllten Mikrokristallen oder Mikrokörnchen, und andererseits einer Gesamtheit von Füllstoffen, die ein Zerfallsmittel, ein lösliches Mittel sowie ein Gleitmittel umfaßt;
- das Vermischen des Wirkstoffes und der Füllstoffe, außer mindestens des größten Teils des Gleitmittels;
- das Eintragen einer Menge von dieser Mischung, die notwendig für die Bildung einer Tablette ist, in den Hohlraum einer Preßvorrichtung, in deren Innerem die Mischung gepreßt werden soll, und an deren Wandungen zuvor die erforderliche Menge von Gleitmittel aufgebracht wurde;
- das Verpressen der Mischung und das Auswerfen der gebildeten Tablette.

12. Verfahren nach Anspruch 11, **dadurch gekennzeichnet, daß** die Preßkräfte 3 bis 50 kN, vorzugsweise 4 bis 40 kN und noch bevorzugter 5 bis 25 kN betragen.
